# EUROPÄISCHE PATENTSCHRIFT

(11) **EP 1 681 344 B1**
(45) Veröffentlichungstag und Bekanntmachung des Hinweises auf die Patenterteilung: **14.11.2007**
(21) Anmeldenummer: 06000099.9
(22) Anmeldetag: 04.01.2006
(51) Int. Cl.: C12M 1/107, C12M 1/113

(54) **Feststoffbeschickungsvorrichtung an einem Fermenter einer Biogasanlage**
Apparatus for loading solids in a fermenter of a biogas plant
Dispositif de chargement de matière solide d'un fermenteur d'une installation de biogaz

(30) Priorität: 14.01.2005 DE 102005001783
(43) Veröffentlichungstag der Anmeldung: 19.07.2006
(73) Patentinhaber: U.T.S. Umwelt-Technik-Süd GmbH, 84419 Obertaufkirchen (DE)
(72) Erfinder: Bürger, Adam, 83527 Haag (DE)
(74) Vertreter: Liebl, Thomas

(56) Entgegenhaltungen:
- EP-A- 0 803 568
- EP-A- 0 934 998
- EP-A- 1 170 357
- EP-A- 1 251 165
- DE-A1- 2 535 756
- DE-A1- 3 717 725
- DE-A1- 10 022 056
- DE-A1- 10 110 861
- DE-A1- 10 351 529

## Beschreibung

Die Erfindung betrifft eine Biogasanlagen-Feststoffbeschickungsvorrichtung an einem Fermenter einer Biogasanlage nach dem Oberbegriff des Anspruchs 1.

Eine gattungsgemäße Biogasanlagen-Feststoffbeschickungsvorrichtung für einen Fermenter einer Biogasanlage ist bereits aus der EP 1 251 165 A1 bekannt. Konkret weist diese Biogasanlagen-Feststoffbeschickungsvorrichtung einen Misch- und Vorratsbehälter auf, der auf Wägestäben gelagert ist. Das Biomasse-Feststoffschüttgut wird hier in loser Form in den Misch- und Vorratsbehälter eingebracht, um mittels der Wägeeinrichtung verschiedenste organische Feststoffe zu einem genau definierten Biomasse-Substratmix zusammenmischen zu können. Andererseits ist mittels dieser Wägeeinrichtung auch eine genau auf den Fermentationsprozess abgestimmte Menge an Biomasse bei Bedarf chargenweise in den Fermenterbehälter zudosierbar. Die Biomasse wird dabei über eine Zudosierschnecke, die gasdicht in den Fermenterbehälterinnenraum geführt wird, in den Fermenterbehälterinnenraum eingebracht.

Des weiteren ist es üblich, die losen Biogas-Feststoffe als loses Schüttgut in sogenannten Fahr- oder Hochsilos auf Vorrat zu lagern, aus denen heraus die Biomasse dann mittels Ladern etc. zum Fermenterbehälter transportiert wird und dort beispielsweise in den zuvor genannten Misch- und Vorratsbehälter eingebracht wird. Die Silotechnik selbst ist relativ aufwendig.

Aufgabe der Erfindung ist es, eine alternative Biogasanlagen-Feststoffbeschickungsvorrichtung für einen Fermenter einer Biogasanlage zu schaffen, mit der die Zudosierung von Biomasse-Feststoffen in einen Fermenterbehälter einer Biogasanlage weitestgehend automatisiert auf einfache und effiziente Weise mit reduzierten Kosten für die Lagerhaltung möglich ist.

Diese Aufgabe wird gelöst mit den Merkmalen des Patentanspruchs 1.

Gemäß Anspruch 1 weist die Biomasse-Zudosiervorrichtung eine mittels der Steuereinrichtung ansteuerbare Fördereinrichtung auf, auf der zur Biomassespeicherung eine solche Mehrzahl von Biomasse-Feststoffen in komprimierter Form aufweisenden und jeweils ein bestimmtes, vorgegebenes Gewicht aufweisenden Biomasse-Feststoffballen anordenbar ist, die der für einen vorgegebenen Zudosier-Zeitraum dem Fermenterbehälter zudosierbaren Biomasse-Feststoffmenge entspricht. Dieser Fördereinrichtung ist eine mittels der Steuereinrichtung ansteuerbare Feststoffballen-Zerkleinerungseinrichtung nachgeschaltet, der die Biomasse-Feststoffballen mittels der Fördereinrichtung in Abhängigkeit von den vorgegebenen Zudosierparametern bzw. Fermentationsparametern zur Zerkleinerung derselben insbesondere einzeln gesteuert zuführbar sind und die wenigstens eine Biomasse-Auslassöffnung aufweist, über die die zerkleinerten Biomasse-Feststoffe aus der Feststoffballen-Zerkleinerungseinrichtung ausbringbar und dem Fermenterbehälter zuführbar sind.

Besonders vorteilhaft in Verbindung mit einem hohen Automatisierungsgrad und damit auch einem wirtschaftlichen Betreiben der Biogasanlage ist hier, dass die Fördereinrichtung, z. B. eine Bandfördereinrichtung, eine Kettenfördereinrichtung oder eine Rollenfördereinrichtung, in einer Doppelfunktion gleichzeitig als Biomassespeicher dient, da auf der Fördereinrichtung eine solche Mehrzahl von Biomasse-Feststoffballen angeordnet werden kann, die der für den vorgegebenen Zudosier-Zeitraum dem Fermenterbehälter zudosierbaren Biomasse-Feststoffmenge entspricht. Dadurch wird sichergestellt, dass die Biogasanlagen-Feststoffbeschickungsvorrichtung für den vorgegebenen Zeitraum voll automatisiert betrieben werden kann. Beispielsweise ist hier eine solche Mehrzahl von Biomasse-Feststoffballen auf der Fördereinrichtung angeordnet, die der für einen Zeitraum von z. B. 6 bis 48 Stunden dem Fermenterbehälter zudosierbaren Biomasse-Feststoffmenge entspricht.

Besonders vorteilhaft ist es hierbei, wenn die auf der Fördereinrichtung für den vorgegebenen Zudosier-Zeitraum angeordneten Biomasse-Feststoffballen jeweils ein innerhalb vorgebbarer Toleranzbereiche liegendes gleiches Gewicht aufweisen, da mit derartigen, ein in etwa gleiches Gewicht aufweisenden Biomasse-Feststoffballen eine besonders einfache und mittels der elektrischen Steuereinrichtung automatisierte, genaue Zudosierung von Biomasse-Feststoffen in einen Fermenterbehälter einer Biogasanlage auf einfache Weise auch ohne aufwendige Wägeeinrichtungen möglich wird, da über eine einfache Steuerung der Biogasanlagen-Feststoffbeschickungsvorrichtung mittels einer elektrischen Steuereinrichtung auf einfache Weise sichergestellt werden kann, zu welchem Zeitpunkt ein Biomasse-Feststoffballen in die Feststoffballen-Zerkleinerungseinrichtung eingeführt und dort zerkleinert werden muss. So kann beispielsweise für eine Biogasanlage mit einer elektrischen Leistung von 0,5 MW vorgegeben sein, dass bei einem vorgegebenen Biomasse-Feststoffballengewicht von z. B. 700 kg durch entsprechende Ansteuerung der Steuereinrichtung pro Stunde ein Biomasse-Feststoffballen in den Fermenterbehälter zudosiert werden muss. Grundsätzlich könnten aber auch bei eventuell unterschiedlichen Ballengewichten der einzelnen Feststoffballen Wägemittel z. B. an der Fördereinrichtung vorgesehen sein, um das jeweilige Ballengewicht gegebenenfalls in Verbindung mit einer Regelungseinrichtung zu erfassen.

Hierbei hat es sich, wie das eben genannte Zahlenbeispiel zeigt, weiterhin als besonders vorteilhaft erwiesen, dass das Gewicht der Biomasse-Feststoffballen in Abhängigkeit von Fermentationsparametern als Zudosierparametern vorgegeben ist, und zwar vorzugsweise so vorgegeben ist, dass das Ballengewicht der zu einem vorgegebenen Zudosierzeitpunkt während des Zudosier-Zeitraums zuzudosierenden Biomasse-Feststoffmenge entspricht. Für den oben genannten Beispielfall, bei dem mittels der Biogasanlagen-Feststoffbeschickungsvorrichtung jede Stunde eine Biomasse-Feststoffmenge von 700 kg in den Fermenterbehälter zudosiert werden muss, beträgt daher das Ballengewicht vorteilhaft 700 kg. In Verbindung mit diesem Beispiel wäre es grundsätzlich aber auch möglich, Biomasse-Feststoffballen mit einem Gewicht von in etwa 350 kg vorzugeben. In einem solchen Fall müssten dann jede Stunde entsprechend zwei Biomasse-Feststoffballen mit der Fördereinrichtung in die Feststoffballen-Zerkleinerungseinrichtung gefördert und dort entsprechend zerkleinert bzw. als dann genau zudosierte Biomasse-Feststoffcharge in den Fermenterbehälter eingebracht werden.

Nach der Zerkleinerung des Biomasse-Feststoffballens in der Feststoffballen-Zerkleinerungseinrichtung werden dann die zerkleinerten Biomasse-Feststoffe über die Biomasse-Auslassöffnung der Feststoffballen-Zerkleinerungseinrichtung aus dieser ausgebracht und anschließend dem Fermenterbehälter als Biomasse-Feststoffcharge zugeführt. Die Ausbringung der zerkleinerten Biomasse-Feststoffe aus der Feststoffballen-Zerkleinerungseinrichtung kann dabei auf vielfältige Art und Weise vorgenommen werden. So ist grundsätzlich auch ein - bezogen auf die in der Feststoffballen-Zerkleinerungseinrichtung vorhandene zerkleinerte Biomasse-Feststoffmenge - chargenweises Ausbringen derselben durch entsprechend vorgegebene Ansteuerung einer die Biomasse-Auslassöffnung verschließenden Verschlusseinrichtung möglich, die in Abhängigkeit von z. B. der Öffnungszeit die Ausbringung einer bestimmten Biomasse-Feststoffmenge ermöglicht. Grundsätzlich wäre es dabei auch möglich, z. B. in Verbindung mit der Feststoffballen-Zerkleinerungseinrichtung Wägemittel vorzusehen. Ein besonderer Vorteil der Erfindung liegt jedoch gerade darin, dass diese Wägeeinrichtung aufgrund der ein bestimmtes, vorgegebenes Gewicht aufweisenden Biomasse-Feststoffballen grundsätzlich eingespart werden können, so dass eine insgesamt einfache sowie kostengünstige und wirtschaftlich betreibbare Biogasanlagen-Feststoffbeschickungsvorrichtung an einem Fermenter einer Biogasanlage zur Verfügung gestellt wird.

Mit der Kompaktierung zu Biomasse-Feststoffballen, bei der die Biomasse-Feststoffe nicht mehr in loser Schüttgutform vorliegen, können zudem auch die Lagerhaltungskosten für Biomasse-Feststoffe grundsätzlich reduziert werden: So können diese Biomasse-Feststoffballen einfach und kostengünstig gelagert werden, z. B. im Freien, da sie im Gegensatz zu Schüttgut z. B. nicht gegen Winderosion zu schützen sind, so dass die Bereitstellung von Schüttgut-Silos entfallen kann, wobei derartige Biomasse-Feststoffballen zudem einfachst übereinander gestapelt werden können. Aufgrund der Komprimierung der Biomasse-Feststoffe kann gegenüber dem losen Schüttgut auch der Lager- bzw. Stauraum - bezogen auf eine jeweils gleiche Menge Biomasse - auch insgesamt vorteilhaft reduziert werden. Derartige Biomasse-Feststoffballen lassen sich durch z. B. aus der Landwirtschaft bekannte Pressen relativ einfach mit definiertem Gewicht herstellen. Das Ballengewicht liegt vorzugsweise zwischen 150 bis 1500 kg, wobei je nach Ballengewicht unterschiedliche Toleranzbereiche vorgegeben werden können. So liegen beispielsweise die Toleranzbereiche bei Ballengewichten von 150 bis 400 kg bei ca. ± 10 bis 20 kg, während bei Ballengewichten von 400 kg bis 1000 kg Toleranzen von ± 50 kg vorgegeben werden können. Bei Ballengewichten über 1000 kg können auch Toleranzbereiche von bis zu 100 kg vorgegeben werden.

Die Fördereinrichtung selbst ist z. B. in der Art eines herkömmlichen Förderbandes ausgebildet, so dass die einzelnen Biomasse-Feststoffballen in Förderrichtung hintereinander angeordnet werden können, wobei ein Ende der Fördereinrichtung einer Balleneinwurföffnung der Feststoffballen-Zerkleinerungseinrichtung so zugeordnet ist, dass bei einer Ansteuerung und Betätigung der Fördereinrichtung ein jeweils vorderer Biomasse-Feststoffballen in die Feststoffballen-Zerkleinerungseinrichtung eingebracht bzw. eingeworfen werden kann. Da die Biomasse-Feststoffballen, z. B. in der Art von Strohballen, als Rundballen oder Rechteckballen aus einem organischen Feststoffmaterial ausgebildet sind, die mit Bändern, Schnüren, Hüllen etc. umgeben sind, ist gegebenenfalls vor dem Einbringen der Biomasse-Feststoffballen in die Feststoffballen-Zerkleinerungseinrichtung ein Entfernen dieser Folien, Hüllen, Bänder, Schnüre etc. erforderlich, was z. B. manuell erfolgen kann. Um in einem solchen Fall, z. B. in Verbindung mit Rundballen ein Aufrollen der Biomasse-Feststoffballen zu vermeiden, sind bevorzugt Hilfsmittel an der Fördereinrichtung angeordnet, die diesem Aufrollen entgegenwirken. Z. B. kann dies auf einfache Weise dadurch realisiert werden, dass die Fördereinrichtung quer zur Förderrichtung gesehen um einen vorgegebenen Winkel gegen die Horizontale geneigt ist, so dass das freie Ende des Biomasse-Feststoffbands bei seiner entsprechenden Anordnung auf der Fördereinrichtung mit seinem Gewicht in Richtung auf ein Abstütz- und/oder Führungsmittel an der Fördereinrichtung gepresst wird, was dem Aufrollen entgegenwirkt. Diese Abstütz- und/ oder Führungsmittel können zudem eine vorteilhafte Führung der Ballen in Förderrichtung zur Verfügung stellen. Grundsätzlich ist jedoch auch in Verbindung mit der Entfernung von den die Biomasse-Feststoffballen umgebenden Folien, Hüllen, Bändern, Schnüren etc. auch eine Automatisierung dahingehend möglich, dass z. B. im endseitigen Bereich der Fördereinrichtung, und damit entsprechend im Bereich der Feststoffballen-Zerkleinerungseinrichtung eine hier beispielhaft als Schneidvorrichtung bezeichnete Vorrichtung angeordnet ist, mittels der diese Folien, Hüllen, Bänder, Schnüre etc. bei deren Vorbeibewegung durchtrennt und gegebenenfalls auch gesammelt werden können.

Die Ballenzerkleinerungseinrichtung selbst weist ein z. B. trogförmiges, nach oben offenes Gehäuse auf, dessen offene Oberseite die Balleneinwurföffnung ausbildet, der entsprechend ein Ende der Fördereinrichtung zugeordnet ist. In dem Gehäuse der Feststoffballen-Zerkleinerungseinrichtung ist ferner eine Zerkleinerungskammer als Ballenaufnahmeraum ausgebildet, in der Zerkleinerungsmittel, wie z. B. Schneid- und/oder Reißkanten aufweisende Schnecken als Zerkleinerungsmittel so angeordnet ist bzw. sind, dass die zerkleinerten Biomasse-Feststoffe aus der Zerkleinerungskammer zur Biomasse-Auslassöffnung hin und aus dieser heraus förderbar sind. Besonders vorteilhaft ist dies mit einer vorzugsweise mittels einem elektrischen Schneckenantrieb antreibbaren Schnecke möglich. Die Schnecken können dabei sowohl horizontal als auch vertikal in der Zerkleinerungskammer angeordnet sein. Gegebenenfalls sind in diesem Zusammenhang auch weitere Hilfsmittel, wie z. B. Leitbleche in der Zerkleinerungskammer angeordnet, mittels denen der Austrag der zerkleinerten Biomasse-Feststoffe über die Biomasse-Auslassöffnung unterstützt wird. Eine derartige Feststoffballen-Zerkleinerungseinrichtung kann vorteilhaft relativ kleinbauend und kompakt aufgebaut sein und je nach der Anzahl der vorhandenen Zerkleinerungsmittel bzw. Schneid- und/oder Reißkanten aufweisenden Schnecken mit geringem Leistungsaufwand betrieben werden.

Der Biomasse-Auslassöffnung der Feststoffballen-Zerkleinerungseinrichtung ist weiterhin eine Förderschnecke zugeordnet und nachgeschaltet, mittels der die zerkleinerten Biomasse-Feststoffe gasdicht in den Fermenterbehälter förderbar sind. Diese Förderschnecke ist mit einem Schneckengehäuse gasdicht durch eine Fermenterbehälterwand geführt und taucht mit einem Gehäuseendbereich in die Fermenterflüssigkeit ein. Beispielsweise weist diese Förderschnecke am der Biomasse-Auslassöffnung zugeordneten Schneckenende eine trichterförmige Erweiterung auf.

Die Erfindung wird nachfolgend anhand einer Zeichnung näher erläutert.

Es zeigen:
- Fig. 1: eine schematische Darstellung einer erfindungsgemäßen Biogasanlagen-Feststoffbeschickungsvorrichtung an einem Fermenter einer Biogasanlage,
- Fig. 2: eine schematische Seitenansicht der Feststoffballen-Zerkleinerungseinrichtung,
- Fig. 3: eine schematische Draufsicht auf die Feststoffballen-Zerkleinerungseinrichtung nach Fig. 2,
- Fig. 4: eine schematische Schnittdarstellung entlang der Linie Z-Z der Fig. 1,
- Fig. 5: eine schematische Draufsicht auf einen als Rundballen ausgebildeten Biomasse-Feststoffballen, und
- Fig. 6: eine weitere schematische Darstellung der Biogasanlagen-Feststoffbeschickungsvorrichtung an einem Fermenter einer Biogasanlage nach Fig. 1 mit zusätzlicher Zudosiermöglichkeit von losen Feststoffen.

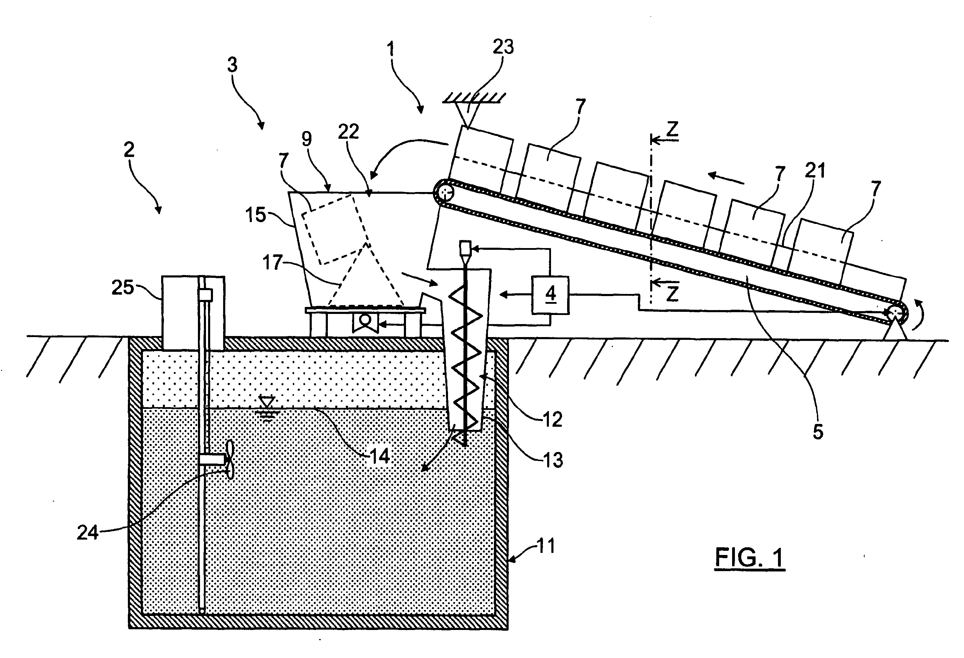

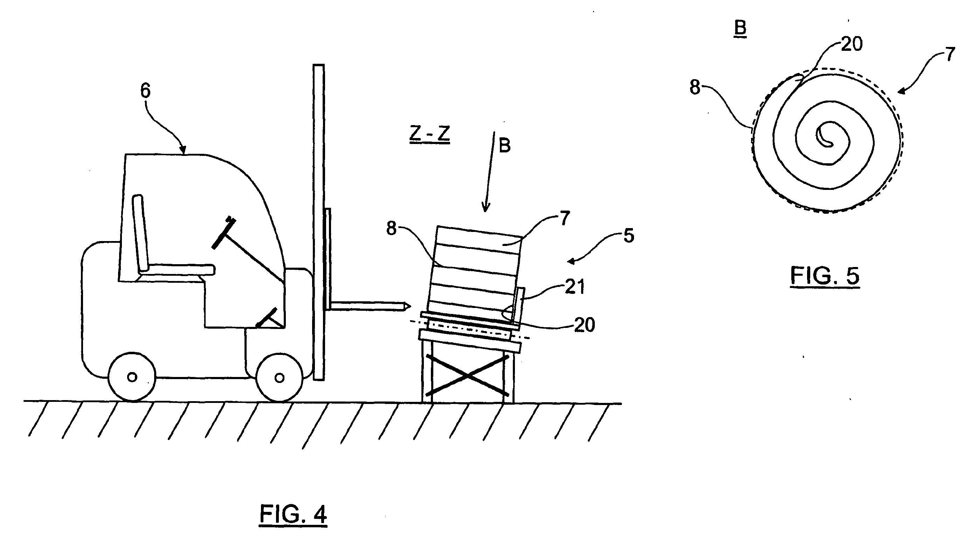

In der Fig. 1 ist schematisch eine Biogasanlagen-Feststoffbeschickungsvorrichtung 1 an einem Fermenter 2 einer hier nur ausschnittweise dargestellten Biogasanlage 3 gezeigt. Diese Biogasanlagen-Feststoffbeschickungsvorrichtung 1 weist als Biomasse-Zudosiervorrichtung ein mittels einer Steuereinrichtung 4, die hier lediglich schematisch als Blackbox gezeichnet ist und regelmäßig im Kontrollraum einer Biogasanlage untergebracht ist, ansteuerbares Förderband 5 auf, auf das, wie dies auch aus der Fig. 4 ersichtlich ist, die einen Schnitt entlang der Linie Z-Z der Fig. 1 zeigt, z. B. mittels eines Gabelstaplers 6 Biomasse-Feststoffballen 7 angeordnet werden, die der für einen bestimmten vorgegebenen Zudosier-Zeitraum von z. B. einem Tag dem Fermenterbehälter chargenweise zudosierbaren Biomasse-Feststoffmenge entspricht.

Das Förderband 5 ist hier so ausgelegt, dass die einzelnen Biomasse-Feststoffballen 7 in Förderrichtung gesehen der Reihe nach hintereinander angeordnet sind.

Die Biomasse-Feststoffballen weisen hier ein jeweils innerhalb bestimmter Toleranzbereiche liegendes gleiches Gewicht auf, z. B. 700 kg mit einem Toleranzbereich von ± 10 kg. Ferner sind die Biomasse-Feststoffballen 7, wie dies aus der Fig. 5 ersichtlich ist, z. B. als sog. Rundballen ausgebildet, bei denen ein komprimiertes Biomasse-Feststoffband schneckenartig aufgewickelt wird und anschließend mittels Schnüren bzw. Bändern 8 in seiner runden Form fixiert wird.

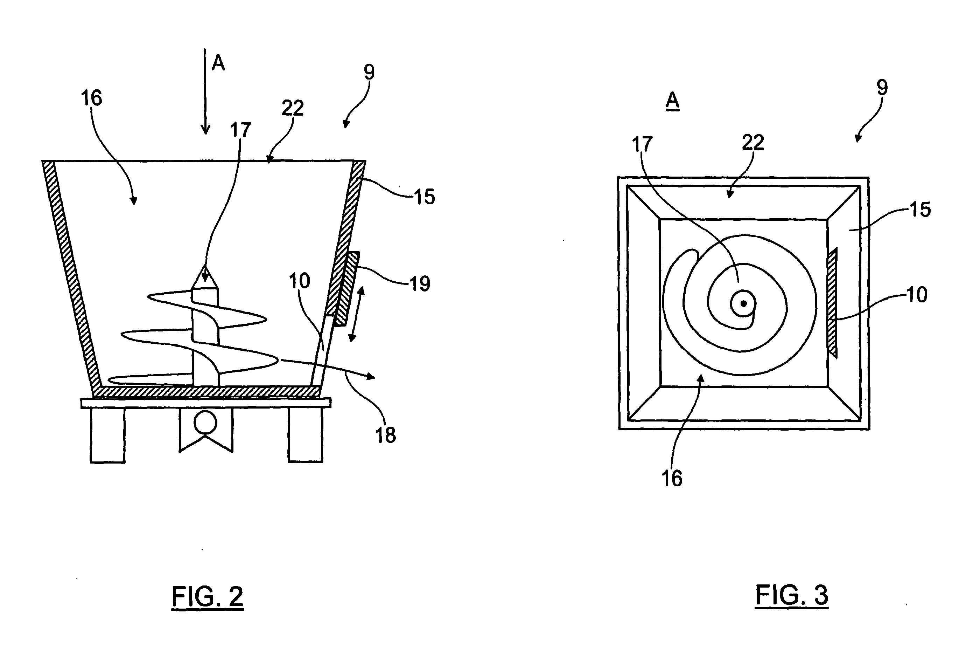

Das Gewicht dieser Biomasse-Feststoffballen 7 ist hier vorteilhaft zudem in Abhängigkeit von Fermentationsparametern als Zudosierparametern vorgegeben, in Verbindung mit dem zuvor genannten Beispiel eines Biomasse-Feststoffballens 7 mit einem Gewicht von 700 kg so vorgegeben, dass bezogen auf eine stündlich erforderliche Biomasse-Zudosierung von 700 kg jede Stunde ein Biomasse-Feststoffballen 7 mittels der Steuereinrichtung 4 in eine dem Förderband 5 nachgeschaltete Feststoffballen-Zerkleinerungseinrichtung 9 gefördert und dort zerkleinert wird, sowie anschließend die dann zerkleinerten Biomasse-Feststoffe über eine in der Fig. 2 im Detail dargestellte Biomasse-Auslassöffnung 10 aus der Feststoffballen-Zerkleinerungseinrichtung 9 ausgebracht und einem Fermenterbehälter 11 des Fermenters 2 mittels einer Förderschnecke 12 als Biomasse-Feststoffcharge zugeführt wird. Dementsprechend wäre das Förderband 5 für den ebenfalls zuvor genannten Fall, dass eine Tagesration an Biomasse-Feststoffen vorgehalten werden soll, so auszulegen, dass 24 dieser jeweils 700 kg aufweisenden Biomasse-Feststoffballen 7 darauf angeordnet werden können. Dies ist in der Fig. 1 aus Übersichtlichkeitsgründen nicht dargestellt. In der Fig. 1 sind einschließlich des bereits "eingeworfenen" Feststoffballens 7 insgesamt lediglich 7 Biomasse-Feststoffballen 7 auf dem Förderband 5 vorgesehen gewesen, was im Lichte des zuvor genannten Beispiels bedeuten würde, dass mittels dieser Biogasanlagen-Feststoffbeschickungsvorrichtung 1 ein automatisierter Betrieb von 7 Stunden aufrechterhalten werden kann.

Die Förderschnecke 12 weist hier an einem oberen Ende eine trichterförmige Erweiterung auf, die der Biomasse-Auslassöffnung 10 zugeordnet ist bzw. mit dieser gekoppelt ist. Das Gehäuse 13 der Förderschnecke 12 ist ferner mit einem unteren Ende gasdicht durch eine Fermenterbehälterwand bis in den Bereich unterhalb des Flüssigkeitsspiegels 14 in den Fermenterbehälterinnenraum geführt, um ein insgesamt gasdichtes Zudosieren von zerkleinerten Biomasse-Feststoffen zu gewährleisten.

Auch die Förderschnecke 12 ist mittels der Steuereinrichtung 4 ansteuerbar.

Die in den Fig. 2 und 3 dargestellte Feststoffballen-Zerkleinerungseinrichtung ist mittels eines hier nicht im Detail dargestellten elektrischen Antriebs, der ebenfalls über die Steuereinrichtung 4 angesteuert wird, antreibbar, wobei dieser Antrieb eine in einem trogförmigen Gehäuse 15 in einer Zerkleinerungskammer 16 angeordnete Vertikalschnecke 17 als Zerkleinerungsmittel antreibt, die hier lediglich äußerst schematisch und beispielhaft dargestellt ist und an den Schneckenrandbereichen z. B. auch Reiß- und/oder Schneidkanten aufweist. Der Antrieb der Vertikalschnecke 17 erfolgt dabei so, dass, wie dies in der Fig. 2 mit dem Pfeil 18 dargestellt ist, die zerkleinerten Biomasse-Feststoffe zur Biomasse-Auslassöffnung 10 hin gefördert werden. Die Biomasse-Auslassöffnung 10 selbst ist mittels einer hier beispielhaft als Schieber 19 ausgebildeten Verschlusseinrichtung verschließbar, die in Abhängigkeit von einem Steuersignal der Steuereinrichtung 4 zur Freigabe der Biomasse-Auslassöffnung 10 ansteuerbar ist. Die Ansteuerung des Schiebers 19 kann dabei auch so erfolgen, dass damit ein chargenweises Ausbringen von in der Feststoffballen-Zerkleinerungseinrichtung 9 vorliegenden zerkleinerten Biomasse-Feststoffen erfolgt, was gegebenenfalls auch durch entsprechende Schneckenbetätigung unterstützt werden kann. Grundsätzlich ist jedoch auch ein Aufbau denkbar, bei dem die Biomasse-Auslassöffnung 10 dauerhaft offen ist und die zerkleinerten Biomasse-Feststoffe während bzw. nach dem Zerkleinern von Biomasse-Feststoffballen 7 sukzessive für eine vorgegebene Zeit aus der Feststoffballen-Zerkleinerungseinrichtung 9 in Richtung Förderschnecke 12 gefördert werden und als Feststoffcharge in den Fermenterbehälter eingebracht werden. Beispielsweise kann dies unter Berücksichtigung des zuvor genannten Zahlenbeispiels bedeuten, dass bei einer jede Stunde erfolgenden Zerkleinerung eines Biomasse-Feststoffballens 7 über die Biomasse-Auslassöffnung 10 die 700 kg betragende Menge an zerkleinerten Biomasse-Feststoffen während eines Zeitraums von z. B. 15 Minuten ausgetragen wird.

Um insbesondere in Verbindung mit Rundballen als Biomasse-Feststoffballen 7 zu vermeiden, dass diese nach einem manuellen Lösen der Schnüre 8 sich abwickeln bzw. aufrollen, kann das Förderband 5, wie dies in der Fig. 4 gezeigt ist, so gegen die Horizontale geneigt sein, dass ein freies Ende 20 des Biomasse-Feststoffballens 7 auf der tiefer liegenden Seite des Förderbandes 5 gegen eine am Förderband 5 ausgebildete Stützwand 21 gedrückt wird, so dass dieser Abrolltendenz entgegengewirkt wird, da dann der Biomasse-Feststoffballen 7 mit einem Großteil seines Gewichts auf das freie Ende 20 drückt. Die Stützwand 21 ist bevorzugt mit dem Förderband 5 so verbunden, dass diese in einer weiteren Doppelfunktion gleichzeitig eine Führungsfunktion bei einem Transport der Biomasse-Feststoffballen 7 in Förderrichtung übernimmt.

Wie dies in der Fig. 1 lediglich äußerst schematisch angedeutet ist, kann im Bereich der Balleneinwurföffnung 22 des trogförmigen Gehäuses 15 der Feststoffballen-Zerkleinerungseinrichtung 9 bzw. im Endbereich des Förderbandes 5 eine Schneidvorrichtung 23 angebracht sein, mittels der die Schnüre 8 automatisiert durchtrennbar sind. Die Schneidvorrichtung 23 kann auch an anderer Stelle entlang des Förderweges des Förderbandes 5 angeordnet sein, ebenso wie auch mehrere Schneidvorrichtungen vorgesehen sein können.

Im Fermenterbehälter selbst befindet sich noch ein Rührwerk 24 mit Serviceschacht 25.

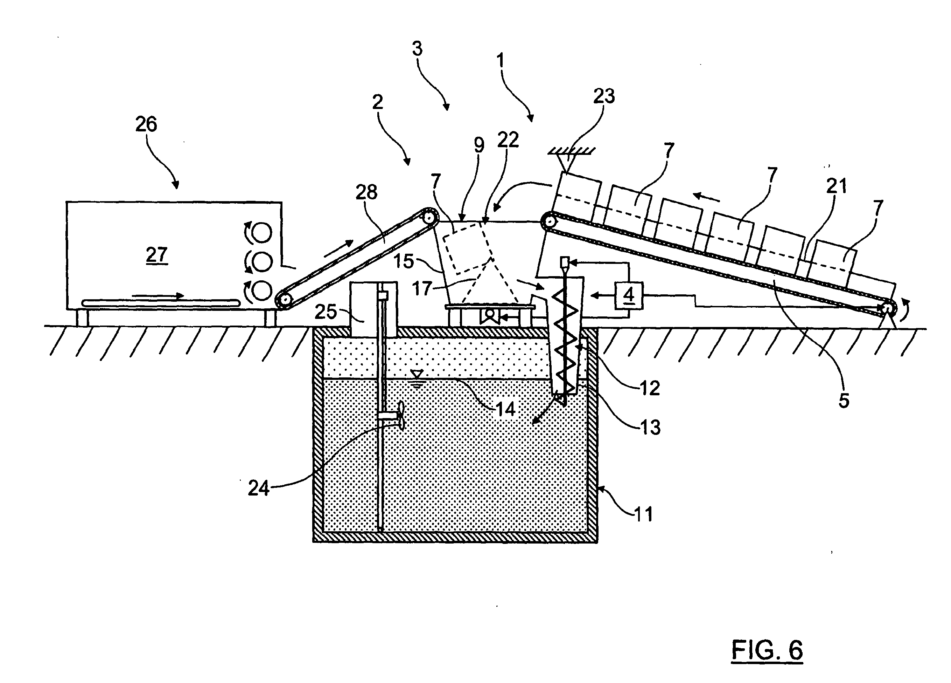

In der Fig. 6 ist schematisch nochmals ein Aufbau entsprechend Fig. 1 gezeigt, jedoch in Kombination mit einer zusätzlichen Beschickungsvorrichtung 26 für lose Biomasse-Feststoffe, die aus einem entsprechenden Vorratsbehälter 27 mittels eines Förderbandes 28 in die Feststoffballen-Zerkleinerungseinrichtung 9 zudosiert werden können. Beispielsweise können hierbei in Abhängigkeit von vorgegebenen Fermentationsparametern lose Feststoffe wie Mais, Grassilage zudosiert werden, falls dies erforderlich sein sollte.

## Patentansprüche

1. Biogasanlagen-Feststoffbeschickungsvorrichtung für einen Fermenter einer Biogasanlage,
mit einer Steuereinrichtung zur Ansteuerung einer Biomasse-Zudosiervorrichtung zum in Abhängigkeit von vorgegebenen Zudosierparametern insbesondere chargenweisen Zudosieren von Biomasse-Feststoffen in einen Fermenterbehälter,
**dadurch gekennzeichnet,**
**dass** die Biomasse-Zudosiervorrichtung eine mittels der Steuereinrichtung (4) ansteuerbare Fördereinrichtung (5) aufweist, auf der zur Ausbildung eines Biomassespeichers eine solche Mehrzahl von Biomasse-Feststoffe in komprimierter Form aufweisenden Biomasse-Feststoffballen (7) eines bestimmten, vorgebbaren Gewichts anordenbar ist, die der für einen vorgegebenen Zudosier-Zeitraum dem Fermenterbehälter (11) insbesondere chargenweise zudosierbaren Biomasse-Feststoffmenge entspricht, und
**dass** der Fördereinrichtung (5) eine mittels der Steuereinrichtung (4) ansteuerbare Feststoffballen-Zerkleinerungseinrichtung (9) nachgeschaltet ist, der die Biomasse-Feststoffballen (7) mittels der Fördereinrichtung (5) in Abhängigkeit von den vorgegebenen Zudosierparametern zur Zerkleinerung derselben gesteuert zuführbar sind und die wenigstens eine Biomasse-Auslassöffnung (10) aufweist, über die die zerkleinerten Biomasse-Feststoffe aus der Feststoffballen-Zerkleinerungseinrichtung (9) ausbringbar und dem Fermenterbehälter (11) zuführbar sind.

2. Biogasanlagen-Feststoffbeschickungsvorrichtung nach Anspruch 1, **dadurch gekennzeichnet, dass** die auf der Fördereinrichtung (5) für einen vorgegebenen Zudosier-Zeitraum angeordneten Biomasse-Feststoffballen (7) jeweils ein innerhalb vorgebbarer Toleranzbereiche liegendes gleiches Gewicht aufweisen.

3. Biogasanlagen-Feststoffbeschickungsvorrichtung nach Anspruch 1 oder Anspruch 2, **dadurch gekennzeichnet, dass** das Gewicht der Biomasse-Feststoffballen (7) in Abhängigkeit von Fermentationsparametern als Zudosierparametern vorgegeben ist, vorzugsweise so vorgegeben ist, dass das Gewicht eines Biomasse-Feststoffballens (7) einer zu einem vorgegebenen Zudosierzeitpunkt während des Zudosier-Zeitraums zuzudosierenden Biomasse-Feststoffmenge entspricht.

4. Biogasanlagen-Feststoffbeschickungsvorrichtung nach einem der Ansprüche 1 bis 3, **dadurch gekennzeichnet, dass** die Fördereinrichtung (5) so zu einem vorgegebenen Zudosierzeitpunkt während des Zudosier-Zeitraums mittels der Steuereinrichtung (4) ansteuerbar ist, dass eine vorgegebene Anzahl von Biomasse-Feststoffballen (7), insbesondere von ein innerhalb vorgebbarer Toleranzbereiche ein gleiches Gewicht aufweisenden Biomasse-Feststoffballen (7), in die Feststoffballen-Zerkleinerungseinrichtung (9) gefördert und dort zerkleinert wird, und dass anschließend die zerkleinerten Biomasse-Feststoffe über die Biomasse-Auslassöffnung (10) aus der Feststoffballen-Zerkleinerungseinrichtung (9) ausbringbar und dem Fermenterbehälter (11) als Biomasse-Feststoffcharge zuführbar sind.

5. Biogasanlagen-Feststoffbeschickungsvorrichtung nach einem der Ansprüche 1 bis 4, **dadurch gekennzeichnet, dass** die zerkleinerten Biomasse-Feststoffe aus der Feststoffballen-Zerkleinerungseinrichtung (9) in Abhängigkeit von vorgegebenen Zudosierparametern mittels der Steuereinrichtung (4) zu vorgebbaren Zudosierzeitpunkten gesteuert chargenweise über die Biomasse-Auslassöffnung (10) ausbringbar sind.

6. Biogasanlagen-Feststoffbeschickungsvorrichtung nach einem der Ansprüche 1 bis 5, **dadurch gekennzeichnet, dass** die Biomasse-Feststoffballen (7) als Rundballen oder Rechteckballen aus einem organischen Feststoffmaterial ausgebildet sind.

7. Biogasanlagen-Feststoffbeschickungsvorrichtung nach einem der Ansprüche 1 bis 6, **dadurch gekennzeichnet, dass** die wenigstens eine Biomasse-Auslassöffnung (10) mittels einer mittels der Steuereinrichtung (4) ansteuerbaren Verschlusseinrichtung (19), vorzugsweise einem Schieber und/oder einer Schwenkklappe, verschließbar ist.

8. Biogasanlagen-Feststoffbeschickungsvorrichtung nach einem der Ansprüche 1 bis 7, **dadurch gekennzeichnet, dass** die Feststoffballen-Zerkleinerungseinrichtung (9) ein vorzugsweise trogförmig ausgebildetes Gehäuse (15) mit einer über eine Balleneinwurföffnung (22) zugänglichen Zerkleinerungskammer (16) aufweist, in der wenigstens ein Zerkleinerungsmittel (17) und gegebenenfalls weitere Hilfsmittel so angeordnet ist bzw. sind, dass die zerkleinerten Biomasse-Feststoffe aus der Zerkleinerungskammer (16) zur Biomasse-Auslassöffnung (10) hin und aus dieser heraus förderbar sind.

9. Biogasanlagen-Feststoffbeschickungsvorrichtung nach Anspruch 8, **dadurch gekennzeichnet, dass** als Zerkleinerungsmittel wenigstens eine Schneid- und/oder Reißkanten aufweisende und mit einem vorzugsweise elektrischen Schneckenantrieb antreibbare Schnecke (17) vorgesehen ist, die in der Zerkleinerungskammer (16) vertikal oder horizontal angeordnet ist.

10. Biogasanlagen-Feststoffbeschickungsvorrichtung nach einem der Ansprüche 1 bis 9, **dadurch gekennzeichnet, dass** der Biomasse-Auslassöffnung (10) eine Förderschnecke (12) zugeordnet und nachgeschaltet ist, mittels der die zerkleinerten Biomasse-Feststoffe gasdicht in den Fermenterbehälter (11) förderbar sind.

11. Biogasanlagen-Feststoffbeschickungsvorrichtung nach Anspruch 10, **dadurch gekennzeichnet, dass** die Förderschnecke (12) mit einem Schneckengehäuse (13) gasdicht durch eine Fermenterbehälterwand geführt ist und mit einem Gehäuseendbereich in die Fermenterflüssigkeit (14) eintaucht.

12. Biogasanlagen-Feststoffbeschickungsvorrichtung nach einem der Ansprüche 1 bis 11, **dadurch gekennzeichnet, dass** auf der einen Biomassespeicher ausbildenden Fördereinrichtung (5) eine solche Mehrzahl von Biomasse-Feststoffballen (7) anordenbar ist, die der für einen Zudosier-Zeitraum von 6 bis 48 Stunden, bevorzugt von 12 bis 24 Stunden dem Fermenterbehälter (11) zudosierbaren Biomasse-Feststoffmenge entspricht.

13. Biogasanlagen-Feststoffbeschickungsvorrichtung nach einem der Ansprüche 1 bis 12, **dadurch gekennzeichnet, dass** die Fördereinrichtung (5), vorzugsweise eine Bandfördereinrichtung oder eine Kettenfördereinrichtung oder eine Rollenfördereinrichtung, so ausgebildet ist, dass die einzelnen Biomasse-Feststoffballen (7) in Förderrichtung hintereinander angeordnet sind, und
dass ein Ende der Fördereinrichtung (5) einer Balleneinwurföffnung (22) der Feststoffballen-Zerkleinerungseinrichtung (9) so zugeordnet ist, dass bei einer Ansteuerung und Betätigung der Fördereinrichtung (4) ein jeweils vorderer Biomasse-Feststoffballen (7) in die Feststoffballen-Zerkleinerungseinrichtung (9) einbringbar ist.

14. Biogasanlagen-Feststoffbeschickungsvorrichtung nach einem der Ansprüche 1 bis 13, **dadurch gekennzeichnet, dass** die Fördereinrichtung (5) Abstütz- und/oder Führungsmittel (21) aufweist, die die Biomasse-Feststoffballen quer zur Förderrichtung gesehen wenigstens bereichsweise und wenigstens auf einer Seite abstützen, und/oder in Förderrichtung führen.

15. Biogasanlagen-Feststoffbeschickungsvorrichtung nach Anspruch 13 und 14, **dadurch gekennzeichnet, dass** die Fördereinrichtung (5) quer zur Förderrichtung gesehen um einen vorgegebenen Winkel gegen die Horizontale geneigt und die Abstütz- und/oder Führungsmittel (21) wenigstens auf der tiefer liegenden Seite angeordnet sind.

16. Biogasanlagen-Feststoffbeschickungsvorrichtung nach einem der Ansprüche 1 bis 15, **dadurch gekennzeichnet, dass** die Fördereinrichtung (5) eine, vorzugsweise dort endseitig und vorzugsweise einer Balleneinwurföffnung (22) zugeordnete Schneidvorrichtung (23) aufweist, mittels der die Biomasse-Feststoffballen (7) umgebenden Folien und/oder Hüllen und/oder Bänder und/oder Schnüre (8) durchtrennbar sind.

17. Verfahren zum Betreiben einer Biogasanlagen-Feststoffbeschickungsvorrichtung an einem Fermenter einer Biogasanlage nach einem der Ansprüche 1 bis 16, **dadurch gekennzeichnet, dass** eine entsprechend einem vorgegebenen Zudosier-Zeitraum vorgegebene Mehrzahl von komprimierten Biomasse-Feststoffballen (7) auf einer Fördereinrichtung (4) angeordnet wird,
dass bei einer Ansteuerung der Fördereinrichtung (4) eine vorgegebene Anzahl von Biomasse-Feststoffballen (7) in eine Feststoffballen-Zerkleinerungseinrichtung (9) eingebracht und dort zerkleinert wird, und
dass anschließend die zerkleinerten Biomasse-Feststoffe aus der Feststoffballen-Zerkleinerungseinrichtung (9) ausgebracht und insbesondere als Biomasse-Feststoffcharge in einen Fermenterbehälter (11) zudosiert werden.

## Claims

1. Apparatus for loading solids in a fermenter of a biogas plant,
with a control device for activating a metered biomass feeding device for the metered feeding of biomass solids into a fermenter container in dependence on predetermined metered feeding parameters, in particular in batches,
**characterized**
**in that** the metered biomass feeding device has a conveying device (5), which can be activated by means of the control device (4) and on which such a plurality of bales of solid biomass material (7), comprising biomass solids in compressed form of a specific, predeterminable weight, that corresponds to the amount of biomass solids that can be fed in a metered manner, in particular in batches, to the fermenter container (11) for a predetermined metered feeding period can be arranged to form a biomass store, and
**in that** the conveying device (5) is followed downstream by a solid bale comminuting device (9), which can be activated by means of the control device (4) and to which the bales of solid biomass material (7) can be fed in a controlled manner by means of the conveying device (5) in dependence on the predetermined metered feeding parameters for comminuting the same, and which has at least one biomass outlet opening (10), via which the comminuted biomass solids can be discharged from the solid bale comminuting device (9) and can be fed to the fermenter container (11).

2. Apparatus for loading solids in a biogas plant according to Claim 1, **characterized in that** the bales of solid biomass material (7) arranged on the conveying device (5) for a predetermined metered feeding period are each of a weight that is the same within predeterminable tolerance ranges.

3. Apparatus for loading solids in a biogas plant according to Claim 1 or Claim 2, **characterized in that** the weight of the bales of solid biomass material (7) is predetermined in dependence on fermentation parameters as metered feeding parameters, preferably is predetermined in such a way that the weight of a bale of solid biomass material (7) corresponds to an amount of solid biomass material that is to be fed in a metered manner at a predetermined metered feeding time during the metered feeding period.

4. Apparatus for loading solids in a biogas plant according to one of Claims 1 to 3, **characterized in that** the conveying device (5) can be activated at a predetermined metered feeding time during the metered feeding period by means of the control device (4) in such a way that a predetermined number of bales of solid biomass material (7), in particular of bales of solid biomass material (7) that are of the same weight within predeterminable tolerance ranges, are conveyed into the solid bale comminuting device (9) and comminuted there, and **in that** subsequently the comminuted biomass solids can be discharged from the solid bale comminuting device (9) via the biomass outlet opening (10) and can be fed to the fermenter container (11) as a batch of solid biomass material.

5. Apparatus for loading solids in a biogas plant according to one of Claims 1 to 4, **characterized in that** the comminuted biomass solids can be discharged in a controlled manner from the solid bale comminuting device (9) in dependence on predetermined metered feeding parameters by means of the control device (4) at predeterminable metered feeding times in batches via the biomass outlet opening (10).

6. Apparatus for loading solids in a biogas plant according to one of Claims 1 to 5, **characterized in that** the bales of solid biomass material (7) are formed as round bales or rectangular bales of an organic solid material.

7. Apparatus for loading solids in a biogas plant according to one of Claims 1 to 6, **characterized in that** the at least one biomass outlet opening (10) can be closed by means of a closure device (19) that can be activated by means of the control device (4), preferably a slide and/or a hinged flap.

8. Apparatus for loading solids in a biogas plant according to one of Claims 1 to 7, **characterized in that** the solid bale comminuting device (9) has a housing (15) of a preferably trough-shaped form, with a comminuting chamber (16), which is accessible via a bale charging opening (22) and in which at least one comminuting means (17) and possibly further auxiliary means is or are arranged in such a way that the comminuted biomass solids can be conveyed into and out of the comminuting chamber (16) to the biomass outlet opening (10).

9. Apparatus for loading solids in a biogas plant according to Claim 8, **characterized in that** at least one screw (17) is provided as the comminuting means, which screw has cutting and/or tearing edges, can be driven by a preferably electric screw drive and is arranged vertically or horizontally in the comminuting chamber (16).

10. Apparatus for loading solids in a biogas plant according to one of Claims 1 to 9, **characterized in that** the biomass outlet opening (10) is assigned and followed downstream by a conveying screw (12), by means of which the comminuted biomass solids can be conveyed into the fermenter container (11) in a gastight manner.

11. Apparatus for loading solids in a biogas plant according to Claim 10, **characterized in that** the conveying screw (12) is led with a screw housing (13) in a gastight manner through a fermenter container wall and dips with a housing end region into the fermenter liquid (14).

12. Apparatus for loading solids in a biogas plant according to one of Claims 1 to 11, **characterized in that** such a plurality of bales of solid biomass material (7) that corresponds to the amount of biomass solids that can be fed in a metered manner to the fermenter container (11) for a metered feeding period of 6 to 48 hours, with preference of 12 to 24 hours, can be arranged on the conveying device (5) forming a biomass store.

13. Apparatus for loading solids in a biogas plant according to one of Claims 1 to 12, **characterized in that** the conveying device (5), preferably a belt conveying device or a chain conveying device or a roller conveying device, is formed in such a way that the individual bales of solid biomass material (7) are arranged one after the other in the conveying direction, and
**in that** one end of the conveying device (5) is assigned to a bale charging opening (22) of the solid bale comminuting device (9) in such a way that, when the conveying device (5) is activated and actuated, a solid bale of biomass material (7) that is respectively at the front can be introduced into the solid bale comminuting device (9).

14. Apparatus for loading solids in a biogas plant according to one of Claims 1 to 13, **characterized in that** the conveying device (5) has supporting and/or guiding means (21), which support the bales of solid biomass material at least in certain regions and at least on one side, seen transversely in relation to the conveying direction, and/or guide them in the conveying direction.

15. Apparatus for loading solids in a biogas plant according to Claims 13 and 14, **characterized in that** the conveying device (5) is inclined, seen transversely in relation to the conveying direction, by a predetermined angle with respect to the horizontal and the supporting and/or guiding means (21) are arranged at least on the lower-lying side.

16. Apparatus for loading solids in a biogas plant according to one of Claims 1 to 15, **characterized in that** the conveying device (5) has a cutting device (23), which is preferably at the end thereof, is preferably assigned to a bale charging opening (22) and by means of which films and/or wrappings and/or strips and/or cords (8) surrounding the bales of solid biomass material (7) can be severed.

17. Method of operating an apparatus for loading solids in a fermenter of a biogas plant according to one of Claims 1 to 16, **characterized in that** a predetermined plurality of compressed bales of solid biomass material (7) corresponding to a predetermined metering period are arranged on a conveying device (5),
**in that**, when the conveying device (5) is activated, a predetermined number of bales of solid biomass material (7) are introduced into a solid bale comminuting device (9) and comminuted there, and
**in that** subsequently the comminuted biomass solids are discharged from the solid bale comminuting device (9) and are fed in a metered manner into a fermenter container (11), in particular as a batch of solid biomass material.

## Revendications

1. Dispositif de chargement de matière solide d'une installation de biogaz pour un fermenteur d'une installation de biogaz,
avec un dispositif de commande pour le pilotage d'un dispositif d'addition dosée de biomasse pour une addition dosée des matières solides de biomasse dans un récipient de fermenteur, en fonction de paramètres prédéfinis d'addition dosée, en particulier par charges,
**caractérisé en ce que**
le dispositif d'addition dosée de la biomasse présente un dispositif d'alimentation (5) dirigeable à l'aide du dispositif de commande (4), sur lequel peut être agencé, pour la formation d'un réservoir de biomasse, un tel nombre de balles de matière solide de biomasse (7) présentant les matières solides de biomasse sous forme comprimée, d'un poids défini donné, qui correspond à la quantité de matière solide de biomasse pouvant être additionnée de façon dosée pendant une période donnée d'addition dosée du récipient de fermenteur (11), en particulier par charges, et
**en ce que** le dispositif d'alimentation (5) est suivi d'un dispositif de fragmentation (9) des balles de matière solide dirigeable à l'aide du dispositif de commande (4), où les balles de matière solide de biomasse (7) peuvent être amenées de manière dirigée, à l'aide du dispositif d'alimentation (5), en fonction des paramètres prédéfinis d'addition dosée, pour leur fragmentation et qui présente au moins une ouverture de sortie de la biomasse (10), par laquelle la matière solide de biomasse fragmentée peut être prélevée du dispositif de fragmentation des balles de matière solide (9) et peuvent être amenées dans le récipient de fermenteur (11).

2. Dispositif de chargement de matières solides d'une installation de biogaz selon la revendication 1, **caractérisé en ce que** les balles de matière solide de biomasse (7), agencées sur le dispositif d'alimentation (5) pendant une période prédéfinie d'addition dosée, présentent chaque fois un poids similaire se trouvant dans une gamme de tolérance donnée.

3. Dispositif de chargement de matières solides d'une installation de biogaz selon la revendication 1 ou 2, **caractérisé en ce que** le poids des balles de matière solide de biomasse (7) est fixé en fonction des paramètres de fermentation, comme paramètre d'addition dosée, de préférence est fixé de sorte que le poids d'une balle de matière solide de biomasse (7) correspond à une quantité de matière solide de biomasse à additionner de façon dosée à un moment donné d'addition dosée pendant la période d'addition dosée.

4. Dispositif de chargement de matières solides d'une installation de biogaz selon l'une des revendications 1 à 3, **caractérisé en ce que** le dispositif d'alimentation (5) peut être dirigé à un moment donné d'addition dosée pendant la période d'addition dosée, à l'aide du dispositif de commande (4) de sorte qu'un nombre donné de balles de matière solide de biomasse (7), en particulier des balles de matière solide de biomasse (7) présentant un poids similaire au sein d'un intervalle de tolérance fixé, est alimenté dans le dispositif de fragmentation des balles de matière solide (9) et y est fragmenté, et **en ce qu'**ensuite, la matière solide de biomasse fragmentée est prélevée du dispositif de fragmentation des balles de matière solide (9) par l'ouverture de sortie de la biomasse (10) et peut être amenée dans le récipient de fermenteur (11) comme charge de matière solide de biomasse.

5. Dispositif de chargement de matières solides d'une installation de biogaz selon l'une des revendications 1 à 4, **caractérisé en ce que** la matière solide de biomasse fragmentée peut être prélevée du dispositif de fragmentation des balles de matière solide (9) en fonction de paramètres fixés d'addition dosée à l'aide du dispositif de commande (4), aux moments d'addition dosée pouvant être fixés, par charges, par l'ouverture de sortie de la biomasse (10).

6. Dispositif de chargement de matières solides d'une installation de biogaz selon l'une des revendications 1 à 5, **caractérisé en ce que** les balles de matière solide de biomasse (7) sont formées comme des balles rondes ou des balles rectangulaires en un matériau solide organique.

7. Dispositif de chargement de matières solides d'une installation de biogaz selon l'une des revendications 1 à 6, **caractérisé en ce que** la au moins une ouverture de sortie de la biomasse (10) peut être fermée à l'aide d'un dispositif de fermeture (19) dirigeable par le dispositif de commande (4), de préférence un curseur et/ou un clapet pivotant.

8. Dispositif de chargement de matières solides d'une installation de biogaz selon l'une des revendications 1 à 7, **caractérisé en ce que** le dispositif de fragmentation des balles de matière solide (9) présente un boîtier (15) de préférence en forme d'auge, avec une chambre de fragmentation (16) accessible par une ouverture d'introduction des balles (22), dans laquelle au moins un moyen de fragmentation (17) et le cas échéant, d'autres moyens sont agencés de sorte que la matière solide de biomasse fragmentée peut être alimentée dans et hors de ceux-ci, depuis la chambre de fragmentation (16) vers l'ouverture de sortie de la biomasse (10).

9. Dispositif de chargement de matières solides d'une installation de biogaz selon la revendication 8, **caractérisé en ce que** comme moyen de fragmentation, est prévue au moins une hélice (17) présentant des bords coupants et/ou de déchirure et actionnable avec une transmission à vis sans fin de préférence électrique, qui est agencée dans la chambre de fragmentation (16), de manière verticale ou horizontale.

10. Dispositif de chargement de matières solides d'une installation de biogaz selon l'une des revendications 1 à 9, **caractérisé en ce que** l'ouverture de sortie de la biomasse (10) est coordonnée à et suivie d'une hélice d'alimentation (12), avec laquelle la matière solide de biomasse fragmentée peut être alimentée dans le récipient de fermenteur (11), de manière étanche aux gaz.

11. Dispositif de chargement de matières solides d'une installation de biogaz selon la revendication 10, **caractérisé en ce que** l'hélice d'alimentation (12) est conduite par un boîtier d'hélice (13), de manière étanche aux gaz par une paroi du récipient de fermenteur et immergée dans le liquide du fermenteur (14) avec une zone d'extrémité de boîtier.

12. Dispositif de chargement de matières solides d'une installation de biogaz selon l'une des revendications 1 à 11, **caractérisé en ce que** sur le dispositif d'alimentation (5) formant un réservoir de biomasse, peut être agencé un certain nombre de balles de matière solide de biomasse (7), qui correspond à la quantité de matière solide de biomasse pouvant être additionnée de façon dosée au récipient de fermenteur (11) pendant une période d'addition dosée de 6 à 48 heures, de préférence de 12 à 24 heures.

13. Dispositif de chargement de matières solides d'une installation de biogaz selon l'une des revendications 1 à 12, **caractérisé en ce que** le dispositif d'alimentation (5) est formé de préférence comme un dispositif d'alimentation à bande ou un dispositif d'alimentation à chaîne ou un dispositif d'alimentation à rouleaux, de sorte que chaque balle de matière solide de biomasse (7) est agencée l'une après l'autre dans le dispositif d'alimentation, et qu'une extrémité du dispositif d'alimentation (5) est coordonnée à une ouverture d'introduction de balles (22) du dispositif de fragmentation des balles de matière solide (9), de sorte que lors d'un pilotage et d'une manoeuvre du dispositif d'alimentation (4), une balle de matière solide de biomasse (7) chaque fois précédente peut être introduite dans le dispositif de fragmentation des balles de matière solide (9).

14. Dispositif de chargement de matières solides d'une installation de biogaz selon l'une des revendications 1 à 13, **caractérisé en ce que** le dispositif d'alimentation (5) présente des moyens d'appui et/ou d'alimentation (21), qui appuient les balles de matière solide de biomasse, transversalement à la direction d'alimentation, au moins par zone et au moins sur un côté, et/ou les conduisent en direction d'alimentation.

15. Dispositif de chargement de matières solides d'une installation de biogaz selon les revendications 13 et 14, **caractérisé en ce que** le dispositif d'alimentation (5) est incliné transversalement à la direction d'alimentation, d'un angle donné par rapport à l'horizontale et **en ce que** les moyens d'appui et/ou d'alimentation (21) sont agencés au moins sur le côté couché en bas.

16. Dispositif de chargement de matières solides d'une installation de biogaz selon l'une des revendications 1 à 15, **caractérisé en ce que** le dispositif d'alimentation (5) présente un dispositif de coupe (23), de préférence sur le côté de l'extrémité et de préférence, coordonné à une ouverture d'introduction de balles (22), à l'aide duquel les feuilles et/ou enveloppes et/ou bandes et/ou ficelles (8) enveloppant les balles de matière solide de biomasse (7) peuvent être séparées.

17. Procédé d'exploitation d'un dispositif de chargement de matières solides d'une installation de biogaz sur un fermenteur d'une installation de biogaz selon une des revendications 1 à 16, **caractérisé en ce qu'**un nombre donné, correspondant à une période donnée d'addition dosée, de balles de matière solide de biomasse (7), comprimées, est agencé sur un dispositif d'alimentation (4),
**en ce que** lors d'un pilotage du dispositif d'alimentation (4), un nombre donné de balles de matière solide de biomasse (7) est introduit dans un dispositif de fragmentation des balles de matière solide (9) et y est fragmenté, et
**en ce qu'**ensuite, la matière solide de biomasse fragmentée est extraite du dispositif de fragmentation de balles de matière solide (9) et additionnée de façon dosée à un récipient de fermenteur (11), en particulier comme charge de matière solide de biomasse.
